(19) Europäisches Patentamt
European Patent Office
Office européen des brevets

(11) **EP 2 668 472 B1**

(12) **EUROPÄISCHE PATENTSCHRIFT**

(45) Veröffentlichungstag und Bekanntmachung des Hinweises auf die Patenterteilung:
**30.08.2017 Patentblatt 2017/35**

(21) Anmeldenummer: **12704228.1**

(22) Anmeldetag: **27.01.2012**

(51) Int Cl.:
**A61B 5/00** (2006.01)        **A61B 5/02** (2006.01)
**A61B 5/021** (2006.01)        **A61F 2/82** (2013.01)
**G01F 1/708** (2006.01)        **G01F 1/72** (2006.01)

(86) Internationale Anmeldenummer:
**PCT/EP2012/000380**

(87) Internationale Veröffentlichungsnummer:
**WO 2012/100959 (02.08.2012 Gazette 2012/31)**

(54) **ZYLINDERFÖRMIGE VORRICHTUNG, PULSWELLENGESCHWINDIGKEITSMESSSYSTEM UND VERFAHREN ZUM MESSEN EINER PULSWELLENGESCHWINDIGKEIT**

CYLINDRICAL DEVICE, PULSE WAVE MEASUREMENT SYSTEM AND METHOD FOR MEASURING A PULSE WAVE SPEED

DISPOSITIF CYLINDRIQUE, SYSTÈME DE MESURE DE LA VITESSE DE L'ONDE DE POULS ET PROCÉDÉ DE MESURE DE LA VITESSE DE L'ONDE DE POULS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.01.2011 DE 102011009695**

(43) Veröffentlichungstag der Anmeldung:
**04.12.2013 Patentblatt 2013/49**

(73) Patentinhaber: **Fraunhofer-Gesellschaft zur Förderung der angewandten Forschung e.V.
80686 München (DE)**

(72) Erfinder:
• **DOMNICH, Alexej
71254 Ditzingen (DE)**

• **SCHÄCHTELE, Jonathan
68159 Mannheim (DE)**

(74) Vertreter: **Pfenning, Meinig & Partner mbB
Patent- und Rechtsanwälte
Theresienhöhe 11a
80339 München (DE)**

(56) Entgegenhaltungen:
**WO-A2-2007/067690        WO-A2-2010/057495
US-A1- 2005 080 346**

• **K. TAKAHATA ET AL: "Micromachined Antenna Stents and Cuffs for Monitoring Intraluminal Pressure and Flow", JOURNAL OF MICROELECTROMECHANICAL SYSTEMS, Bd. 15, Nr. 5, 1. Oktober 2006 (2006-10-01), Seiten 1289-1298, XP55028764, ISSN: 1057-7157, DOI: 10.1109/JMEMS.2006.880229**

**Beschreibung**

Die Erfindung betrifft eine zylinderförmige Vorrichtung zum Durchfluss von Fluid, die zumindest zwei Drucksensoren aufweist, mit welchen ein Druck des Fluids im Inneren der Vorrichtung messbar ist. Die Erfindung betrifft außerdem ein Pulswellengeschwindigkeitsmesssystem mit einer solchen zylinderförmigen Vorrichtung, mit welchem die Geschwindigkeit von Pulswellen in der zylinderförmigen Vorrichtung messbar ist. Die Erfindung betrifft weiter ein Verfahren zum Messen der Pulswellengeschwindigkeit in der zylinderförmigen Vorrichtung. Die Erfindung betrifft außerdem ein Verfahren und eine Vorrichtung zum Messen der Elastizität einer Fluidleitung.

In einer Vielzahl von technischen Anwendungen ist es von Interesse, die Geschwindigkeit von Pulswellen in Fluiden zu bestimmen, die durch einen elastischen zylinderförmigen Fluidleiter fließen. Durch die Messung der Pulswellengeschwindigkeit ist es möglich, Verengungen, Verstopfungen oder Verschlüsse in dem Fluidleiter zu detektieren oder auf die Elastizität der Leitung zu schließen.

Ein wichtiges Anwendungsbeispiel sind hierbei beispielsweise Blutgefäße, der Gallengang und ähnliche Gefäße im menschlichen oder tierischen Körper. So kommt es zum Beispiel nach der Implantation von Stents in derartige Gefäße in vielen Fällen (ca. 20 bis 30 %) zu einem Wiederverwachsen mit Gewebe, was zu einem erneuten Verschluss führen kann. Die Folgen können u.a. Herzinfarkt oder Schlaganfall sein. Eine solche Restenose kann bisher nur mit aufwendigen klinischen Messmethoden diagnostiziert werden.

Nach dem Stand der Technik sind zum Erkennen eines solchen Verschlusses regelmäßige Untersuchungen bei einem Arzt, beispielsweise mittels Tonometrie unter Belastung notwendig. Es kann hierbei ein Druck im entsprechenden Gefäß von außen mittels Applanationstonometrie oder invasiv mit Kathetern ("Druckdraht") erfolgen.

Auch bei technischen Leitungen muss der Druck normalerweise von außen oder mittels Sonden gemessen werden.

Journal of Microelectromechanical Systems, Vol. 15, No.5, 2006: "Micromachined Antenna Stets and Cuffs for Monitoring Intraluminal Pressure and Flow" beschreibt ein System mit zwei Microstrukturen, wobei die erste ein induktiver Antennenstent ist, der mit kapazitiven Elementen zur Formung eines Schwingkreises gekoppelt ist. Die andere Struktur ist eine ringförmige Manschette mit zwei Elektroden, die verwendet werden, eine direkte Transduktion der Fließgeschwindigkeit in Gegenwart eines magnetischen Feldes zu ermöglichen.

US 2005/0080346 A1 beschreibt einen Stent, mit dem die Durchlässigkeit eines Gefäßes sichergestellt werden kann und gleichzeitig eine Charakteristik in dem Gefäß überwacht werden kann. Die Vorrichtung enthält eine Struktur mit einer Gruppe von dehnbaren Bändern, die plastisch deformierbar sind und eine Induktivität bilden. Die Struktur weist darüber hinaus eine Kapazität auf, die mit den dehnbaren Bändern verbunden ist und von der Charakteristik im Inneren des Gefäßes abhängig ist.

Es ist daher Aufgabe der vorliegenden Erfindung, eine Vorrichtung anzugeben, mit welcher der Druckverlauf in einem durch die Vorrichtung fließenden Fluid auf einfachere Weise messbar ist. Darüberhinaus soll ein System vorgelegt werden, mit welchem die Geschwindigkeit von Pulswellen in Fluidleitungen einfacher messbar ist. Es sollen darüberhinaus Verfahren zum Messen von Pulswellengeschwindigkeiten in Fluidleitungen außerhalb des menschlichen und tierischen Körpers angegeben werden, die ohne einen Eingriff in Leitung auskommen.

Die Aufgaben werden gelöst durch die zylinderförmige Vorrichtung nach Anspruch 1, das Pulswellengeschwindigkeitsmesssystem nach Anspruch 6, das Verfahren zum Messen einer Pulswellengeschwindigkeit nach Anspruch 14, das Verfahren zum Messen der Elastizität einer Fluidleitung nach Anspruch 15 und die Vorrichtung zum Messen der Elastizität einer Fluidleitung nach Anspruch 16. Vorteilhafte Weiterbildungen der zylinderförmigen Vorrichtung, des Pulswellengeschwindigkeitsmesssystems und des Verfahrens zum Messen einer Pulswellengeschwindigkeit werden durch die jeweiligen abhängigen Ansprüche gegeben.

Im Folgenden wird die Erfindung für die Vorrichtung und die Verfahren gemeinsam beschrieben. Die beschriebenen Merkmale können also im erfindungsgemäßen Verfahren entsprechend angewandt werden.

Erfindungsgemäß wird zunächst eine zylinderförmige Vorrichtung zum Durchfluss von Fluid vorgestellt. Dass die Vorrichtung zylinderförmig sei, bedeutet hierbei, dass sie eine von parallelen Linien, insbesondere Geraden, gebildete Zylinderfläche aufweist. Die Zylinderform wird in den meisten Fällen eine Kreiszylinderform sein, sie kann jedoch erfindungsgemäß auch jede andere Zylinderform umfassen. Möglich sind also auch beispielsweise Zylinder mit dreieckigem, rechteckigem oder vieleckigem Querschnitt.

Die erfindungsgemäße zylinderförmige Vorrichtung kann in eine Fluidleitung einbringbar sein, sie kann aber auch Bestandteil einer Fluidleitung sein oder einen Abschnitt einer Fluidleitung darstellen. Die erfindungsgemäße zylinderförmige Vorrichtung kann bevorzugt elastisch sein und/oder die Fluidleitung kann elastisch sein.

Die zylinderförmige Vorrichtung kann beispielsweise auch eine Fluidleitung, insbesondere eine Flüssigkeitsleitung oder eine Gasleitung, ein Stent zur Implantation im menschlichen oder tierischen Körper, ein Implantat oder ein Katheter sein.

Unter einem Fluid wird erfindungsgemäß eine Substanz verstanden, die einer beliebig langsamen Scherung keinen Widerstand entgegensetzt, die also eine endliche Viskosität hat. Insbesondere sind Gase und Flüssigkeiten Fluide im Sinne dieses Dokuments.

Erfindungsgemäß ist vorgesehen, dass das Fluid die zylinderförmige Vorrichtung entlang einer Zylinderachse der Vorrichtung durchfließt. Die Vorrichtung weist zumindest zwei Drucksensoren auf, die an unterschiedlichen Stellen entlang der Zylinderachse angeordnet sind. In Richtung der Zylinderachse können die Drucksensoren also hintereinanderliegen. Die Drucksensoren können in gleichem Abstand von der Zylinderachse angeordnet sein. Es ist möglich, aber nicht wesentlich, dass die Drucksensoren in der gleichen Richtung von der Zylinderachse aus gesehen angeordnet sind.

Mit den so angeordneten Drucksensoren ist an unterschiedlichen Stellen entlang der Zylinderachse ein Druck des Fluids im Inneren der Vorrichtung messbar. Die Stelle, an welcher der Druck messbar ist, ist normalerweise jene Stelle, an welcher der entsprechende Drucksensor angeordnet ist.

Erfindungsgemäß sind nun die Drucksensoren kapazitive Drucksensoren. Kapazitive Drucksensoren weisen zumindest eine Kapazität bzw. einen Kondensator auf. Die Kapazität ändert sich in Abhängigkeit vom Druck, mit welchem der Drucksensor beaufschlagt wird.

Die Kapazität der Drucksensoren ist mit jeweils einer Spule zu jeweils einem Schwingkreis elektrisch gekoppelt. Jeder Drucksensor bildet also zusammen mit jeweils einer Spule jeweils einen Schwingkreis. Die Schwingkreise unterschiedlicher Drucksensoren sind vorzugsweise voneinander getrennt.

Erfindungsgemäß sind nun die jeweiligen Schwingkreise mittels zumindest eines elektromagnetischen Feldes zum Schwingen anregbar. Die Schwingkreise können hierzu jeweils eine Antenne haben oder Bereiche, die als Antenne wirken. Das elektromagnetische Feld kann hierbei ein elektromagnetisches Wechselfeld oder eine elektromagnetische Welle sein, das mit der Resonanzfrequenz des entsprechenden Schwingkreises schwingt oder eine Schwingung mit dieser Resonanzfrequenz als Komponente aufweist. Es sei darauf hingewiesen, dass das elektromagnetische Feld nicht notwendigerweise ein Wechselfeld sein muss, sondern beispielsweise auch den zeitlichen Verlauf eines Pulses haben kann, der ein kontinuierliches Frequenzspektrum hat. Das kontinuierliche Frequenzspektrum des Pulses sollte dann die Resonanzfrequenzen der anzuregenden Schwingkreise enthalten.

Die erfindungsgemäße Vorrichtung kann vollständig passiv ausgebildet sein. Sie kommt also ohne Spannungsquellen aus und kann ausschließlich mit passiver Elektronik wie Spulen und Kondensatoren realisiert werden. Anders als beispielsweise bei einem Transponder sind keine aktiven Elemente notwendig. Hierdurch hat die erfindungsgemäße Vorrichtung den Vorteil, dass sie sehr klein und zuverlässig realisiert werden kann.

In der erfindungsgemäßen Vorrichtung und dem erfindungsgemäßen Verfahren ist mit den Drucksensoren der Zeitpunkt einer Druckänderung am Ort der Druckmessung bzw. des jeweiligen Drucksensors feststellbar. Eine solche Druckänderung kann insbesondere durch das Passieren einer Druckwelle bzw. Pulswelle am Ort der Messung hervorgerufen werden.

Um aus den gemessenen Zeitpunkten des Passierens der Pulswelle an den Drucksensoren die Laufzeit der Pulswelle von dem einen Drucksensor zum anderen Drucksensor bestimmen zu können, sind die Zeitpunkte der Druckänderung vorteilhafterweise mit einer zeitlichen Auflösung feststellbar, die feiner ist als die Laufzeit der Pulswelle vom einen zum anderen Sensor. In den meisten Ausgestaltungen der Erfindung wird der Druck zu diskreten Zeitpunkten gemessen werden. Zur Messung der Laufzeit der Pulswelle und damit zur Bestimmung der Geschwindigkeit der Pulswelle ist es vorteilhaft, wenn die Zeitpunkte der Druckmessung dichter zusammenliegen als die Zeit, die die Pulswelle benötigt, um vom einen zum anderen Sensor zu laufen. Bevorzugterweise kann der Druck mit einer Frequenz von $\geq 100$ Hz, vorzugsweise $\geq 500$ Hz, besonders bevorzugt $\geq 1000$ Hz gemessen werden.

Beispielsweise angenommen, im Arbeitspunkt beträgt die Pulswellengeschwindigkeit 10m/s und es sollen Änderungen von 1m/s zuverlässig detektiert werden können. Dann lässt sich die notwendige Abtastfrequenz abschätzen mit 1,1 kHz.

Bei der Messung des Druckes wird ausgenutzt, dass die Resonanzfrequenz eines Schwingkreises von der Kapazität und der Induktivität abhängt, die zum Schwingkreis gekoppelt sind. Verändert sich also die Kapazität, so verändert sich auch die Resonanzfrequenz des Schwingkreises. Aus einer Messung der Veränderung der Resonanzfrequenz kann also auf eine Veränderung des Druckes geschlossen werden.

Bevorzugterweise haben die Schwingkreise unterschiedlicher Drucksensoren der zylinderförmigen Vorrichtung unterschiedliche Resonanzfrequenzen, so dass sie unabhängig voneinander zum Schwingen anregbar sind. Da sich die Resonanzfrequenzen durch Änderungen des Druckes verschieben können, liegen die Resonanzfrequenzen unterschiedlicher Schwingkreise bevorzugt so weit auseinander, dass sie sich für keine der auftretenden Drücke überlappen.

Erfindungsgemäß wird auch ein Pulswellengeschwindigkeitsmesssystem bereitgestellt, dessen Bestandteil die vorstehend beschriebene zylinderförmige Vorrichtung ist. Das Pulswellengeschwindigkeitsmesssystem weist darüberhinaus außerdem zumindest eine Auswerteeinheit auf, mit der das elektromagnetische Feld erzeugbar ist, mittels welchem die Schwingkreise zum Schwingen anregbar sind. Dabei ist das elektromagnetische Feld zumindest mit den Frequenzen erzeugbar, bei denen die Schwingkreise bei zumindest einem Druck zum Schwingen anregbar sind. Dieser Druck kann vorteilhaft der Normaldruck in Abwesenheit von Pulswellen sein, so dass also mit der Auswerteeinheit elektromagnetische Felder mit den Frequenzen der Schwingkreise bei Normaldruck erzeugbar sind. Mit der Auswerteeinheit sind nun jene Zeitpunkte bestimmbar, zu welchen die Pulswelle die jeweiligen Sensoren bzw. Messpunkte passiert. Diese Zeitpunkte können dadurch bestimmt werden, dass die Zeitpunkte bestimmt werden, zu denen sich die Resonanz des jeweiligen Schwingkreises ändert. Aus der Differenz der Zeitpunkte des Passierens der Druckwelle an den beiden Drucksensoren und dem Abstand der Drucksensoren zueinander kann dann die Geschwindigkeit der Pulswelle bestimmt werden. Mit dem erfindungsgemäßen Pulswellengeschwindigkeitsmesssystem ist es also möglich, die Pulswellengeschwindigkeit berührungslos und insbesondere ohne in die Fluidleitung eindringen zu müssen, zu messen.

Die Schwingkreise der zylinderförmigen Vorrichtung können als lose gekoppelt mit der Auswerteeinheit angesehen werden. Die Auswerteeinheit kann dabei eine Spule zur Erzeugung des elektromagnetischen Feldes aufweisen.

Die Schwingkreise der Drucksensoren der zylinderförmigen Vorrichtung haben vorzugsweise jeweils eine Antenne oder einen als Antenne wirkenden Bereich, so dass sie mittels des elektromagnetischen Feldes zum Schwingen anregbar sind.

Es bestehen verschiedene Möglichkeiten, durch Messung einer Änderung der Resonanz der Schwingkreise die Zeitpunkte des Passierens einer Pulswelle am Ort eines Drucksensors zu bestimmen, um hieraus die Pulswellengeschwindigkeit zu ermitteln.

In einer Ausgestaltung der Erfindung kann mit der Auswerteeinheit zeitabhängig bestimmbar sein, welcher der Schwingkreise der zylinderförmigen Vorrichtung mit der Resonanzfrequenz bei Normaldruck resoniert. Zeitpunkte, zu denen die Resonanz bei der Resonanzfrequenz bei Normaldruck unterbrochen oder vermindert wird, können dann als Zeitpunkte der Änderung der Resonanz bestimmt werden und damit als Zeitpunkte, zu welchen die Druckwelle den entsprechenden Sensor passiert.

Es kann hierbei besonders vorteilhaft mit der Auswerteeinheit eine Impedanz messbar sein und als Zeitpunkt der Unterbrechung oder Verminderung der Resonanz im oben genannten Sinne einen Zeitpunkt der Verringerung der Impedanz bei der Resonanzfrequenz bei Normaldruck des entsprechenden Schwingkreises bestimmt werden. Es wird hierbei also zeitabhängig die Impedanz des entsprechenden Schwingkreises bei der Resonanzfrequenz gemessen, die der Schwingkreis bei Normaldruck hat. Verringert sich die Impedanz, so deutet dies darauf hin, dass sich die Resonanzfrequenz verschoben hat, was auf eine Druckänderung hinweist.

In einer alternativen Ausführungsform der Erfindung können mittels der Auswerteeinheit elektromagnetische Felder mit variierenden Frequenzen erzeugt werden, wobei die Frequenzen jeweils in einem Bereich variieren, in dem die Resonanzfrequenzen der entsprechenden Schwingkreise variieren können.

Es kann dann für jede der Frequenzen des elektromagnetischen Feldes die Impedanz der Schwingkreise gemessen werden. Die Impedanz weist bei der aktuellen Resonanzfrequenz des jeweiligen Schwingkreises einen lokalen Extremwert, also z.B. einen Peak bzw. eine Spitze, auf. Wird die Frequenz des anregenden elektrischen Feldes also über alle in Frage kommenden Frequenzen variiert und jeweils für die eingestellte Frequenz die Impedanz gemessen, so stellt man bei der aktuellen Resonanzfrequenz des entsprechenden Schwingkreises eine Erhöhung der Impedanz gegenüber den anderen Frequenzen fest.

Es können hierbei die in Frage kommenden Resonanzfrequenzen der zumindest zwei Schwingkreise der Vorrichtung periodisch durchgescannt werden, so dass innerhalb einer Periode des Scans der entsprechende Schwingkreis mit allen in Frage kommenden Frequenzen beaufschlagt wird. Es können hierbei insbesondere kontinuierliche Frequenzintervalle gescannt werden, z.B. mittels eines Wobbel-Generators. Die Frequenzen des elektromagnetischen Feldes werden hierbei mit einer Frequenz durchgescannt, die eine zeitliche Auflösung erlaubt, wie sie zur Bestimmung der Pulswellengeschwindigkeit notwendig ist. Insbesondere sollte also die Dauer des Scannens aller in Frage kommenden Frequenzen deutlich kürzer sein als die Laufzeit der Pulswelle von einem Sensor zum anderen Sensor.

Es sei darauf hingewiesen, dass durch die Auswerteeinheit vorzugsweise alle in der zylinderförmigen Vorrichtung vorhandenen Schwingkreise und Drucksensoren unabhängig voneinander und gleichzeitig auswertbar sind. Es werden also vorzugsweise gleichzeitig Frequenzen zur Anregung des einen und des anderen Schwingkreises erzeugt.

Die entsprechenden Frequenzen aller Schwingkreise können mit einer gemeinsamen Spule erzeugt werden, die mit einer Wechselspannung der entsprechenden Frequenzen beaufschlagt wird. Der Spannungsverlauf und damit der Verlauf des elektromagnetischen Feldes ist dann hierbei die Überlagerung der zu einem gegebenen Zeitpunkt an die Schwingkreise anzulegenden Frequenzen im Sinne einer Fouriersynthese.

In einer weiteren Ausgestaltung der Erfindung kann mittels der Auswerteeinheit ein pulsförmiges elektromagnetisches Feld zur Anregung der Schwingkreise erzeugbar sein. Im Sinne einer Fourieranalyse ist ein pulsförmiges elektromagnetisches Feld ein Feld mit einer Überlagerung von Frequenzen eines kontinuierlichen Frequenzspektrums. Der Puls weist also alle Frequenzen innerhalb eines bestimmten Frequenzbereichs auf.

Dieser Frequenzbereich kann dabei so gewählt sein, dass er alle Resonanzfrequenzen der in der zylinderförmigen Vorrichtung vorkommenden Schwingkreise umfasst, es ist aber auch möglich, dass der Frequenzbereich für jeden Schwingkreis einen disjunkten Teilbereich enthält.

Es werden nun die Schwingkreise mittels des pulsförmigen elektromagnetischen Feldes zum Schwingen angeregt. Nach dem Ende des Pulses werden dann mittels der Auswerteeinheit jene elektromagnetischen Felder gemessen, welche die Schwingkreise beim Abklingen aussenden. Die Frequenz dieser elektromagnetischen Felder entspricht gerade der Resonanzfrequenz der gedämpften Schwingkreise, aus welcher der aktuelle Druck oder eine gegenwärtige Druckänderung bestimmbar ist.

Wird das beschriebene pulsförmige elektromagnetische Feld durch die Auswerteeinheit wiederholt bzw. periodisch erzeugt, so kann nach jedem Puls die Resonanzfrequenz der Schwingkreise gemessen werden. Passiert nun also die Pulswelle einen der Drucksensoren, ändert sich dessen Kapazität und damit die Resonanzfrequenz des entsprechenden Schwingkreises. Da der Puls zur Anregung alle in Frage kommenden Frequenzen enthält, regt er auch den Schwingkreis mit durch den veränderten Druck veränderter Resonanzfrequenz zum Schwingen an. Während des Abklingvorgangs erzeugt dieser Schwingkreis dann ein elektromagnetisches Feld mit verschobener Frequenz. Der Zeitpunkt einer solchen Frequenzverschiebung kann daher als Zeitpunkt des Passierens der Pulswelle bestimmt werden. Wiederum kann durch Bildung der Differenz der Zeitpunkte des Passierens der Druckwelle an unterschiedlichen Drucksensoren die Pulswellengeschwindigkeit bestimmt werden.

In einer weiteren Ausgestaltung der Erfindung kann beispielweise mittels eines Signalgenerators ein periodisches Signal mit konstanter Frequenz erzeugt werden. Die Frequenz wird dabei so gewählt, dass sie für beide Schwingkreise bzw. für beide Sensoren einem Druck entspricht, der im Verlauf einer Pulswelle von dem Druckverlauf durchlaufen wird. Für die Schwingkreise beider Sensoren gilt:

$$f_{res} = \frac{1}{2\pi\sqrt{L \cdot CS}}$$

wobei CS eine Funktion des Druckes p an den kapazitiven Sensoren ist. CS ist gerade die Kapazität des entsprechenden Sensors. Über den oben beschriebenen Zusammenhang ist jedem Druck p eine Resonanzfrequenz $f_{res}$ zugeordnet.

Die beschriebene konstante Frequenz des eingestrahlten elektromagnetischen Signals entspricht also für jeden Sensorschwingkreis einem konstanten Druck. Dieser kann vorteilhaft für die Schwingkreise beider Sensoren identisch sein.

Vorteilhaft kann nun die Frequenz so gewählt werden, dass der entsprechende Druck, bei welchem die Schwingkreise resonieren, zwischen minimalem und maximalem Druck des von einer Pulswelle durchlaufenen Druckverlaufes liegt. Die konstante Funktion bzw. der konstante Druck wird also so von der Druckkurve der Pulswellen während einer Pulswelle mindestens zweimal geschnitten. Im Moment des Schneidens wird der Schwingkreis mit seiner Resonanzfrequenz angeregt.

Die Resonanz kann auf Seiten des das periodische Signal mit konstanter Frequenz sendenden Senders festgestellt werden. Dies kann beispielsweise durch Beobachtung der Änderung der Impedanz des Senders geschehen, die sich aufgrund der induktiven Kopplung zwischen Sender und Sensorschwingkreisen ergibt. Wird daher beispielsweise die Spannung vorgegeben, kann die Impedanzänderung durch Änderung des Stromflusses im Senderschwingkreis festgestellt werden.

Vorteilhafterweise kann der Strom gemessen werden und das Messsignal durch einen Filter mit Tiefpasswirkung geführt werden. Hierdurch lassen sich im Moment des Schneidens Stromänderungen erkennen.

Der zeitliche Abstand zweier gleicher Phasen der Pulswellen, entsprechend den Schnitten des konstanten Druckes, ist gerade der zeitliche Abstand der Detektion der entsprechenden Phasen der Pulswelle an den unterschiedlichen Sensoren. Der zeitliche Abstand ist also gerade die Pulswellenlaufzeit von einem Sensor zum anderen Sensor, aus welcher die Pulswellengeschwindigkeit als Abstand der Sensoren geteilt durch Pulswellenlaufzeit bestimmbar ist.

Bevorzugterweise wird die konstante Frequenz des gesendeten Signals so gewählt, dass die Druckkurve im Verlaufe einer Pulswelle den entsprechenden Druck nur zweimal pro Puls schneidet.

Vorteilhafterweise kann die Einstellung der Frequenz vor der eigentlichen Messung automatisch erfolgen, indem die Frequenz des eingestrahlten elektromagnetischen Feldes solange variiert wird, bis während der Dauer zwischen zwei Pulsen genau zwei Schnitte detektiert werden, also detektiert wird, dass jeder Puls den entsprechenden Druck genau zweimal schneidet. Zur automatischen Einstellung der Frequenz ist es vorteilhaft, wenn die Pulsfrequenz bekannt ist oder mittels herkömmlicher Messvorrichtung bestimmt wird.

Die vorstehend beschriebenen erfindungsgemäßen Verfahren, die mit dem erfindungsgemäßen Pulswellengeschwindigkeitsmesssystem durchführbar sind, sind vorzugsweise an technischen Flüssigkeitsleitungen und Flüssigkeitsleitungen außerhalb des menschlichen und tierischen Körpers durchführbar.

Wurde die Pulswellengeschwindigkeit wie vorstehend beschrieben bestimmt, so kann aus dieser auch auf eine Elastizität der Flüssigkeitsleitung, in der sich die Pulswelle ausbreitet, geschlossen werden. Erfindungsgemäß sind daher auch ein Verfahren und eine Vorrichtung zum Messen der Elastizität einer Fluidleitung.

Die Drucksensoren und Schwingkreise gemäß der Erfindung können so ausgestaltet sein, dass sie absolute Druckwerte messen. Hierzu können durch eine Kalibrierung die Resonanzfrequenzen Druckwerten zugeordnet werden. Dies kann auch geschehen, bevor die Drucksensoren in der zylinderförmigen Vorrichtung verbaut werden.

Eine solche absolute Messung ist aber nicht notwendig, da das Passieren der Druckwellen anhand der Änderung des Druckes detektiert wird. Diese kann auch aus den unkalibrierten Resonanzfrequenzen bestimmt werden, ohne dass überhaupt Druckwerte ermittelt werden.

Im Folgenden soll die Erfindung anhand einiger Figuren beispielhaft erläutert werden. Entsprechende Merkmale sind dabei mit gleichen Bezugszeichen versehen. Die in den Beispielen beschriebenen Merkmale können auch unabhängig vom konkreten Beispiel realisiert sein und beliebig unter den Beispielen kombiniert werden.

Es zeigt

Figur 1    eine erfindungsgemäße zylinderförmige Vorrichtung zum Durchfluss von Fluid,

Figur 2    einen zeitlichen Verlauf des Druckes einer Pulswelle,

Figur 3    ein erfindungsgemäßes Pulswellengeschwindigkeitsmesssystem,

Figur 4    eine mögliche elektrische Verschaltung des erfindungsgemäßen Pulswellenmessgeschwindigkeitssystems und

Figur 5    eine Impedanz der erfindungsgemäßen zylinderförmigen Vorrichtung mit Auswerteeinheit für unterschiedliche Frequenzen.

Figur 6    einen Druckverlauf und einen Impedanzverlauf bei Durchgang einer Druckwelle in einer beispielhaften Ausführungsform der Erfindung

Figur 7    eine beispielhafte Schaltung einer Auswerteeinheit

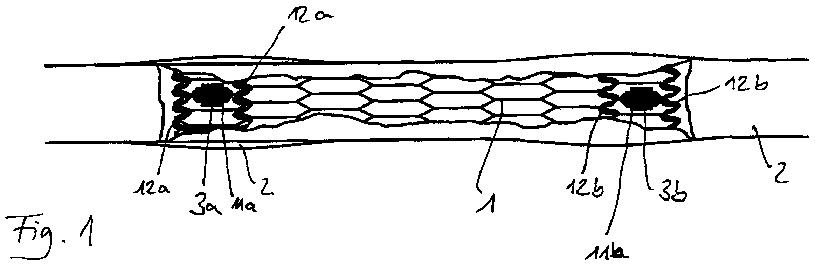

Figur 1 zeigt eine erfindungsgemäße zylinderförmige Vorrichtung zum Durchfluss von Fluid.

Im in Figur 1 gezeigten Beispiel ist die Zylinderförmige Vorrichtung 1 ein Stent 1, der in einem Blutgefäß 2 angeordnet ist. Der Stent 1 weist zwei Drucksensoren 3a und 3b auf, die an unterschiedlichen Stellen entlang einer Zylinderachse des Stents 1 angeordnet sind und mit denen an unterschiedlichen Stellen entlang der Zylinderachse der Druck des Fluids im Inneren des Stents 1 messbar ist. Die Drucksensoren 3a, 3b sind als kapazitive Drucksensoren 3a, 3b ausgebildet. Die Kapazitäten dieser Drucksensoren 3a und 3b sind jeweils mit einer Induktivität 12a, 12b zu jeweils einem Schwingkreis 11a, 11b verbunden. Eigenschaft der kapazitiven Drucksensoren 3a, 3b ist, dass die Kapazitäten der Drucksensoren 3a, 3b vom Druck des Fluids an der Stelle der Messung abhängen. Ändert sich die Kapazität des jeweiligen Drucksensors 3a, 3b, so ändert sich eine Resonanzfrequenz des entsprechenden Schwingkreises. Die Drucksensoren 3a, 3b bzw. die entsprechenden Schwingkreise weisen eine Antenne oder einen als Antenne wirkenden Bereich auf und sind daher mittels eines elektromagnetischen Feldes von außerhalb der Leitung 2 zum Schwingen anregbar.

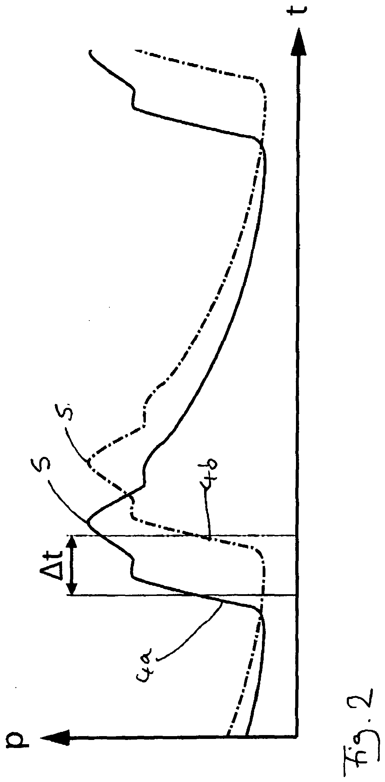

Figur 2 zeigt beispielhaft einen zeitlichen Druckverlauf im Fluid im Inneren des Stents 1. Ein solcher zeitlicher Verlauf kann jeweils durch die Drucksensoren 3a und 3b gemessen werden. Die durchgezogene Linie zeigt den Druckverlauf in einem Drucksensor 3a und die gestrichpunktete Linie den Druckverlauf im anderen Sensor 3b. Zu erkennen ist, dass die Drucksensoren 3a und 3b Druckanstiege 4a, 4b messen, wenn eine Pulswelle 5 den Drucksensor 3a, 3b passiert. Mit den Drucksensoren 3a, 3b ist also ein Zeitpunkt bestimmbar, zu dem die Druckwelle 5 den entsprechenden Sensor 3a, 3b passiert. Zum Festlegen eines Zeitpunktes des Passierens kann beispielsweise ein Schwellenwert festgelegt werden, wobei der Zeitpunkt des Passierens als Zeitpunkt des Überschreitens des Schwellenwertes festgelegt wird. Es ist aber auch z.B. möglich, ein Maximum der Pulswelle 5 zu bestimmen und als Zeitpunkt des Passierens jenen Zeitpunkt zu wählen, zu dem das Maximum den Drucksensor 3a, 3b passiert. Vorteilhaft kann auch das Maximum des Anstiegs der Pulswelle, also das Maximum der ersten Ableitung, zum Festlegen des Zeitpunktes gewählt werden. Eine Vielzahl weiterer Punkte der Druckwelle können zum Festlegen des Zeitpunktes des Passierens gewählt werden. Eine gegebene Druckwelle 5 läuft nun vom Sensor 3a zum Sensor 3b in einer Zeit $\Delta t$. Gemessen werden also jener Zeitpunkt, zu dem der Drucksensor 3a eine bestimmte Phase (beispielsweise das Überschreiten eines Schwellenwertes) misst und der Zeitpunkt, zu dem der andere Drucksensor 3b diese gleiche Phase misst.

Wird nun jener Zeitpunkt bestimmt, zu welchem die Pulswelle 5 den Drucksensor 3a passiert und jener Zeitpunkt, zu welchem die Druckwelle 5 den Sensor 3b passiert, so ist die Zeit $\Delta t$ gerade die Differenz dieser beiden Zeitpunkte. Da der Abstand zwischen den Drucksensoren 3a und 3b bekannt ist, kann nun die Pulswellengeschwindigkeit als Abstand der Drucksensoren 3a und 3b geteilt durch $\Delta t$ bestimmt werden. Aus der Pulswellengeschwindigkeit kann dann beispielsweise auf die Elastizität bzw. Steifigkeit der Leitung 2 geschlossen werden. Generell gilt, dass die Pulswellengeschwindigkeit PWV umso größer ist, je steifer die Leitung 2 ist. Es gilt der Zusammenhang: PWV $\sim$ Zc, wobei Zc, die charakteristische Impedanz der elastischen Leitung, ein Maß für die Steifigkeit ist. Genauer gilt: PWV=A*Zc/rho

A: Querschnittsfläche des Gefäßes, rho: Dichte des Fluids. Es ist also

die     Pulswellengeschwindigkeit     $PWV = \dfrac{A \cdot Z_c}{\rho}$ ,

die     Charakteristische Impedanz des Blutgefäßes     $Z_c = \sqrt{Z_L \cdot Z_T}$ ,

die     Longitudinale Impedanz     $Z_L = \dfrac{\rho}{A}$ und

die     Transversale Impedanz     $Z_T = \dfrac{\partial P}{\partial A}$.

In einer gegebenen Leitung 2 können die gemessenen Werte der Pulswellengeschwindigkeit als Maß für die Elastizität herangezogen werden. Es ist aber auch möglich, die gemessenen Werte der Pulswellengeschwindigkeit auf Absolutwerte der Elastizität zu kalibrieren.

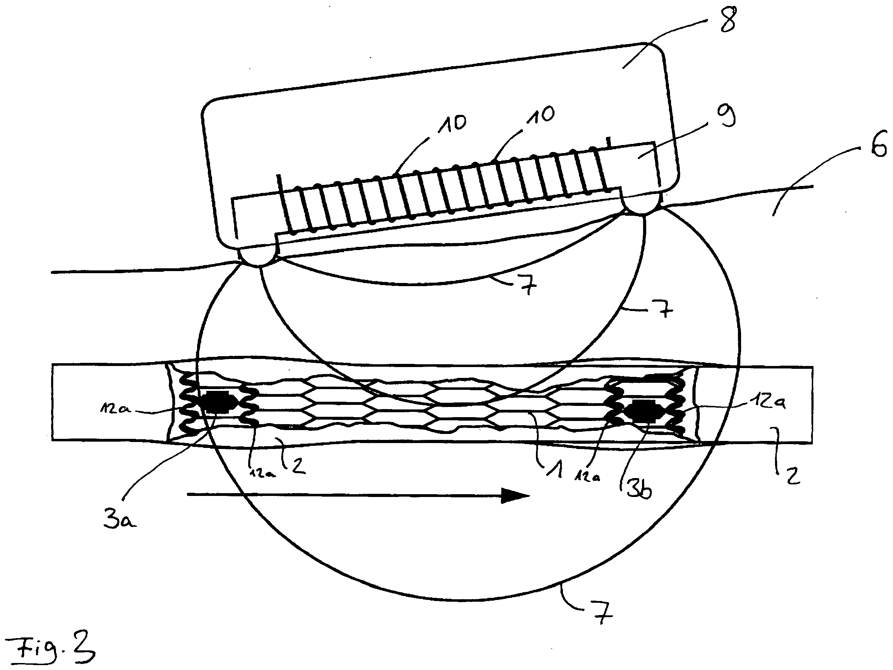

Figur 3 zeigt eine zylinderförmige Vorrichtung 1 gemäß der vorliegenden Erfindung in einer Leitung 2, wie sie in Figur 1 gezeigt ist. Wiederum ist die zylinderförmige Vorrichtung 1 hier ein Stent mit den beiden Drucksensoren 3a und 3b. Die Leitung 2 ist im gezeigten Beispiel wiederum ein Blutgefäß 2, das in einem Körperteil 6 verläuft. Durch eine Auswerteeinheit 8 ist nun ein elektromagnetisches Wechselfeld 7 erzeugbar, mit welchem die Schwingkreise der Drucksensoren 3a, 3b zum Schwingen anregbar sind. Das elektromagnetische Feld enthält hierbei Wechselfelder mit den Resonanzfrequenzen der Schwingkreise der Drucksensoren 3a und 3b. Das elektromagnetische Feld 7 wird in dem in Figur 3 gezeigten Beispiel mittels einer Spule 10 erzeugt, die um einen Kern 9 gewickelt ist. Zur Erzeugung des elektromagnetischen Feldes 7 wird die Spule 10 mit einer Wechselspannung beaufschlagt, die Frequenzkomponenten enthält, die den erforderlichen Frequenzkomponenten des elektromagnetischen Feldes 7 entsprechen. Die Auswerteeinheit 8 bildet zusammen mit der zylinderförmigen Vorrichtung 1 ein erfindungsgemäßes Pulswellengeschwindigkeitsmesssystem.

Die Pulswellengeschwindigkeit ist mit diesem Pulswellengeschwindigkeitsmesssystem auf verschiedene Weisen messbar. Das Verfahren wird hier am Beispiel einer flexiblen Leitung in einer technischen Vorrichtung dargestellt. Wie beschrieben sind mittels des elektromagnetischen Feldes 7 die Schwingkreise der Drucksensoren 3a und 3b zum Schwingen anregbar. Hierzu wird das elektromagnetische Feld 7 mit Resonanzfrequenzen der Schwingkreise erzeugt. Der Zeitpunkt des Passierens einer Pulswelle 5a, 5b an den Sensoren kann nun bestimmt werden, indem eine Änderung der Resonanz des jeweiligen Schwingkreises bestimmt wird. Aus der Differenz der Änderung der Resonanz an den beiden Drucksensoren 3a, 3b kann dann wie oben beschrieben die Pulswellengeschwindigkeit bestimmt werden.

In einem Beispiel des erfindungsgemäßen Verfahrens kann mit der Auswerteeinheit 8 eine Impedanz der Schwingkreise der Drucksensoren 3a und 3b bei der Resonanzfrequenz bei Normaldruck des Fluids in der Leitung 2 gemessen werden. Passiert nun eine Druckwelle einen der Drucksensoren 3a, 3b, so ändert sich die Resonanzfrequenz des entsprechenden Schwingkreises, wodurch die Impedanz des Schwingkreises bei Anregung mit der Resonanzfrequenz bei Normaldruck abfällt. Die Zeitpunkte, zu denen also eine Verringerung der Impedanz bei Anregung mit der Resonanzfrequenz bei Normaldruck, also in Abwesenheit von Pulswellen, festgestellt wird, können als Zeitpunkte des Passierens der Pulswelle gesetzt werden.

In einem alternativen Ausführungsbeispiel des Verfahrens ist es möglich, die Frequenz des elektromagnetischen Feldes 7 in einem Bereich zu variieren, der die für mögliche Drücke des Fluids auftretenden Resonanzfrequenzen der Schwingkreise der Drucksensoren 3a und 3b enthält. Die Frequenz kann dann periodisch variiert werden, so dass innerhalb einer Periode die Schwingkreise jeweils mit allen in Frage kommenden Frequenzen beaufschlagt werden. Es kann nun mittels der Auswerteeinheit 8 gemessen werden, bei welcher dieser Frequenzen die Schwingkreise der Drucksensoren 3a und 3b resonieren. Weicht die so bestimmte Resonanzfrequenz von der Resonanzfrequenz des entsprechenden Schwingkreises bei Normaldruck ab, deutet dies darauf hin, dass eine Pulswelle den entsprechenden Drucksensor 3a, 3b passiert. Der Zeitpunkt des Abweichens der Frequenz von der Resonanzfrequenz bei Normaldruck kann also als Zeitpunkt des Passierens der Pulswelle bestimmt werden. Auch hier können wiederum Schwellen eingesetzt werden, oberhalb derer eine Abweichung der Resonanzfrequenz von der Resonanzfrequenz bei Normaldruck als den Zeitpunkt des Passierens gesetzt wird.

In einer weiteren Ausgestaltung des erfindungsgemäßen Verfahrens ist es möglich, mittels der Auswerteeinheit 8 ein elektromagnetisches Feld mit einem breitbandigen Frequenzspektrum zu erzeugen, wobei das Frequenzspektrum die in Frage kommenden Resonanzfrequenzen für alle möglichen auftretenden Drücke der Schwingkreise der Drucksensoren 3a und 3b enthält. Nach Abschalten des elektromagnetischen Feldes schwingen die Schwingkreise aus und erzeugen dabei ihrerseits elektromagnetische Wechselfelder mit Frequenzen, die ihren momentanen Resonanzfrequenzen entsprechen. Durch Messung der Frequenzen dieser Felder können also direkt die aktuellen Resonanzfrequenzen der Schwingkreise gemessen werden. Wird diese Messung wiederholt durchgeführt, so sind also Zeitpunkte bestimmbar, zu denen die Resonanzfrequenzen der Schwingkreise von den Resonanzfrequenzen bei Normaldruck abweichen. Überschreitet diese Abweichung einer vorbestimmte Schwelle, so kann der Zeitpunkt dieses Überschreitens als Zeitpunkt des Passierens einer Druckwelle gesetzt werden. Eine Druckwelle, die die erfindungsgemäße Vorrichtung 1 durchläuft, löst ein solches Ereignis an zwei aufeinanderfolgenden Zeitpunkten an den beiden Drucksensoren 3a, 3b aus. Aus der Differenz dieser Zeitpunkte und dem Abstand der Drucksensoren 3a, 3b kann also die Pulswellengeschwindigkeit bestimmt werden.

Das breitbandige elektromagnetische Feld kann beispielsweise als Puls mit einem kontinuierlichen Frequenzspektrum erzeugt werden.

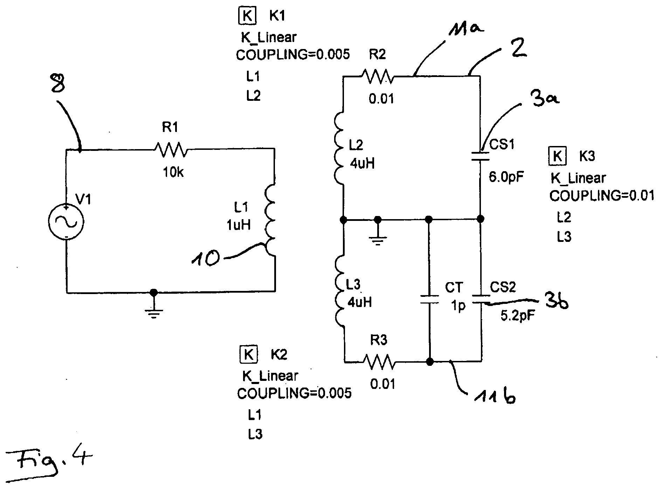

Figur 4 zeigt beispielhaft eine elektrische Schaltung, durch welche das in Figur 2 gezeigte Pulswellenmesssystem realisierbar ist. Hierbei wird in der Auswerteeinheit 8 mittels einer Wechselspannungsquelle V1 an die Spule 10 eine Wechselspannung angelegt, die Frequenzkomponenten enthält, die zur Anregung der Schwingkreise 11a, 11b der Drucksensoren 3a bzw. 3b geeignet sind.

Die kapazitiven Drucksensoren 3a und 3b sind in der Schaltung als Kapazitäten CS1 und CS2 dargestellt. Die Kapazität CS1 des Drucksensors 3a ist mit einer Spule L2 zu einem Schwingkreis gekoppelt. Die Kapazität CS2 des Drucksensors 3b ist mit einer Spule L3 zu einem weiteren Schwingkreis 11b gekoppelt. Die Spule im außerhalb der Leitung, beispielsweise des Blutgefäßes, befindlichen Auslesegeräts 8 wird nachfolgend als Primärspule 10, die Spulen L2, L3 der in dem Blutgefäß befindlichen Sensorschwingkreise als Sekundärspulen bezeichnet.

Für die Sekundärspulen L2, L3 werden in diesem Beispiel zwei rechteckige, flache Luftspulen mit einer Höhe von 5mm, einer Breite von 15mm und einer Windungszahl von 11 gewählt. Nach F.W. Grover, Inductance calculations: working formulas and tables, New York: Dover Pubns, 2004 ergibt sich damit näherungsweise eine Induktivität von L2 = L3 = 4 µH. Bei der Realisierung der Sekundärspulen L2, L3 wird man in der Praxis darauf achten, dass die umschlossene Fläche der Spule so groß wie möglich gewählt wird, um die erreichbare Kopplung zu maximieren.

Die Sekundärspulen L2, L3 werden in diesem Beispiel mit kapazitiven Absolutdrucksensoren 3a, 3b mit einer Grundkapazität von 5 pF und einer Empfindlichkeit von 6 fF/mmHg gekoppelt. Für MEMS-Sensoren in der Größenordnung von 1 mm x 1 mm stellt dies realistische Werte dar. Die Sensoren 3a, 3b besitzen somit eine vom Druck p abhängige Kapazität

$$CS1,2 = 5pF + 6\frac{fF}{mmHg} \cdot p.$$

Die Extremwerte des Ruheblutdrucks liegen bei einem gesunden Menschen im Bereich von 60 mmHg (diastolisch) bis 140 mmHg (systolisch). Da die Sensorik auch für pathologische Drücke geeignet sein soll, wird der Druckmessbereich auf 40 mmHg ≤ p ≤ 180 mmHg festgelegt. Damit ergibt sich für die Kapazität der Drucksensoren

$$5,24pF \leq CS1,2 \leq 6,08pF.$$

Koppelt man einen Sensor direkt mit der oben beschriebenen Spule resultiert als Resonanzfrequenz des Schwingkreises

$$f_{res} = \frac{1}{2\pi\sqrt{L \cdot CS}}:$$

$$32,3MHz \leq f_{res} \leq 34,8MHz.$$

Es ist bekannt, dass die Dämpfung von menschlichem Gewebe um 35 MHz günstig ist, so dass dies einen vorteilhaften Wertebereich darstellt.

Da sich die Frequenzbereiche der beiden Sekundärkreise 11a, 11b nicht überlappen sollen, sollte die Resonanzfrequenz wenigstens eines der beiden Kreise geändert werden. Dies könnte durch eine andere Spule L2, L3 (z.B. andere Windungszahl) geschehen, aber auch durch die Verwendung eines anderen Drucksensors 3a, 3b (z.B. andere aktive Fläche) oder durch das Hinzufügen weiterer Elemente im elektrischen Kreis. In diesem Beispiel wird dem zweiten Sensor 3b eine Kapazität CT parallel geschaltet, um seine Kapazität zu erhöhen und damit die Resonanzfrequenz des zweiten Kreises 11b zu reduzieren. Der zweite Schwingkreis kann durch den hinzugefügten Kondensator CT so verändert werden, dass seine höchste Resonanzfrequenz (für CS2 = 5,24 pF) kleiner 32,3 MHz ist. Der parallel geschaltete Trimmkondensator CT kann somit eine Kapazität von wenigstens 0,82 pF besitzen. Für die Kapazität des Trimmkondensators wird CT = 1 pF gewählt.

Die Ausleseeinheit für die Sensoren kann eine Primärspule 10 besitzen, die mit den Sekundärspulen L2, L3 lose gekoppelt ist. Unter "loser Kopplung" wird verstanden, dass die Koppelimpedanz M sehr viel kleiner als die Wurzel aus dem Produkt der Induktivitäten des Primär- und Sekundärkreises ist, bzw. der Kopplungsfaktor k sehr viel kleiner als 1 ist. Die lose Kopplung hat den gewünschten Nebeneffekt, dass die Kopplung der Kreise sich nicht auf die Resonanzfrequenz auswirkt. Durch die räumliche Entfernung der Spulen in der Praxis kann man in diesem Beispiel von loser Kopplung ausgehen. Im vorliegenden Beispiel hat die Platzierung des Auslesegeräts 8 auf das Messergebnis somit keine Auswirkungen. Voraussetzung ist aber, dass eine ausreichende Kopplung besteht.

Damit die Resonanzen der Empfängerkreise 11a, 11b auf der Primärseite sichtbar werden können, muss außerdem die Eckfrequenz des Primärkreises deutlich größer als die Resonanzfrequenzen der Sensorkreise 11a, 11b sein. Dies wird erreicht, indem der Auslesespule 10 ein Widerstand R1 vorgeschaltet wird. Für die Resonanzfrequenz des Primärkreises ergibt sich

$$f_{res,p} = \frac{R1}{2\pi L1}.$$

In diesem Beispiel wird eine Primärspule 10 mit L1 = 1 $\mu$H verwendet. Diese lässt sich bspw. als Leiterschleife in Luft realisieren. Ein Widerstand von R1 = 10 k$\Omega$ sorgt für eine Grenzfrequenz $f_{res,p}$ = 1,6 GHz.

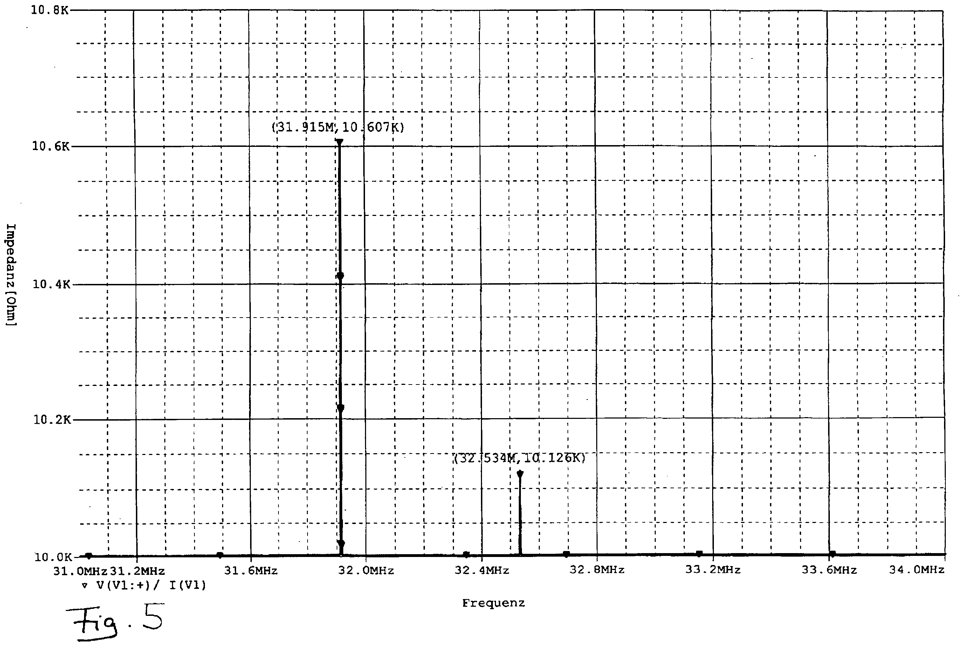

Figur 5 zeigt die Impedanz der Schwingkreise 11a und 11b in Abhängigkeit von der Frequenz des elektromagnetischen Feldes 7. Hierbei wurde als Kopplungsfaktor zwischen dem Primärkreis der Auswerteeinheit 8 und den Sekundärkreisen 11a und 11b jeweils k = 0,005 angenommen. Eine parasitäre Kopplung zwischen den beiden Sekundärkreisen 11a und 11b wurde in Höhe von k = 0,01 berücksichtigt. Die Sekundärspulen L2 und L3 hatten einen Leitungswiderstand von jeweils 10 m$\Omega$. Auf der Primärseite der Auswerteeinheit 8 ergibt sich das in Figur 4 gezeigte Impedanzspektrum. Zu erkennen ist ein Peak bei einer Frequenz von 31,915 MHz mit einer Impedanz von 10,607 k$\Omega$. Ein weiterer Peak ist bei einer Frequenz von 32,534 MHz mit einer Impedanz von 10,126 k$\Omega$ zu erkennen. Aus dem gezeigten Spektrum ist direkt abzulesen, dass der eine Schwingkreis eine Resonanzfrequenz von 31,915 MHz hat und der andere Schwingkreis eine Resonanzfrequenz von 32,534 MHz. Verschieben sich diese Peaks, so deutet dies auf eine Druckänderung hin.

Im Folgenden soll beispielhaft eine Ausführungsform der Erfindung beschrieben werden, in welcher von einem Signalgenerator ein periodisches Signal mit konstanter Frequenz erzeugt wird, die für beide Sensorenkreise einem Druck entspricht, der während einer Pulswelle von der Druckkurve durchlaufen wird. Eine konstante Frequenz entspricht für jeden Sensorkreis einem bestimmten Druck, bei welchem dieser durch die konstante Frequenz zur Resonanz angeregt wird. Dieser Druck kann für beide Sensorkreise identisch sein.

Für die beiden kapazitiven Drucksensoren soll gelten:

$$\boxed{CS1,2 = 5pF + 10\frac{fF}{mmHg} \cdot p}$$

Dabei ist CS die Kapazität des kapazitiven Drucksensors und p der anliegende Druck

Beide Drucksensoren sind mit je einer Spule mit einer Indaktivität L2 = L3 = 4$\mu$H zu einem Schwingkreis gekoppelt.

Beispielhaft durchlaufe der Druck der Pulswelle einen Bereich von 80 bis 120 mmHg an den Sensoren. Die Frequenz des anregenden elektromagnetischen Feldes soll so gewählt werden, dass es in der Mitte zwischen diesen Werten also bei $p_{ref}$ = 100 mmHg liegt. Die Pulswelle schneidet diesen Druck zweimal. Die Kapazität der Sensoren bei diesem Druck ist:

$$\boxed{CS1,2_{ref} = 5pF + 10\frac{fF}{mmHg} \cdot 100mmHg = 6pF}.$$

Damit beträgt die Resonanzfrequenz bei einem Druck von 100mmHg, auf welche der Sender eingestellt wird:

$$f_{res} = \frac{1}{2\pi\sqrt{4uH \cdot 6pF}} = 32,49 MHz$$

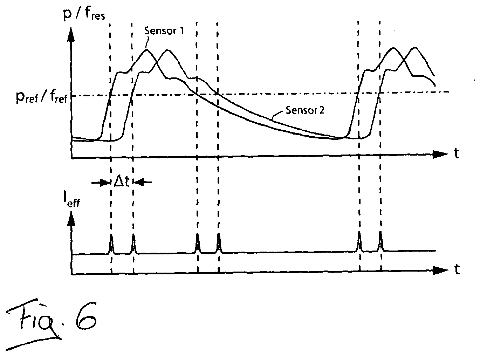

Figur 6 zeigt diese Auswertung beispielhaft. Dabei ist im oberen Teilbild der Druckverlauf an den beiden Sensoren in Abhängigkeit von der Zeit dargestellt und im unteren Teilbild der Verlauf der effektiven Impedanz im Senderschwingkreis zu der im oberen Teilbild gezeigten Zeit.

Durch den Senderschwingkreis wird ein elektromagnetisches Feld mit der Frequenz $f_{ref}$ auf die Sensoren eingestrahlt. Dadurch kommen die Schwingkreise der Sensoren in Resonanz, wenn an den kapazitiven Drucksensoren ein Druck von $p_{ref}$ vorliegt.

Kommt der Sensorschwingkreis in Resonanz, so wird im Sender eine Veränderung der Impedanz gemessen. In Figur 6 ist zu erkennen, dass die Pulswelle den Referenzdruck $p_{ref}$ zunächst am Sensor 1 erreicht und damit zu einem ersten Zeitpunkt eine Veränderung der Impedanz im Senderschwingkreis verursacht, was im unteren Teilbild als Peak zu erkennen ist. Das untere Teilbild zeigt den Effektivwert des Stroms auf der Senderseite, wie er sich durch einen Tiefpassfilter darstellt. Die Impedanz ergibt sich als $Z = \frac{U}{I}$, wobei U die effektive Spannung und I der effektive Strom ist. Nach einer Zeit $\Delta t$ erreicht die Pulswelle und damit der entsprechende Anstieg des Druckes den Sensor 2, so dass der Referenzdruck $p_{ref}$ nun am Sensor 2 vorliegt. Der Sensor 2 gerät zu diesem Zeitpunkt in Resonanz, was im Senderschwingkreis durch einen Anstieg des Stromes erkennbar ist. Im Stromverlauf im unteren Teilbild ist daher nach einer Zeit $\Delta t$ nach dem ersten Peak ein weiterer Peak zu erkennen. Der Abstand $\Delta t$ zwischen den Peaks im Verlauf des Stromes im Senderschwingkreis erlaubt es also unmittelbar, die Laufzeit der Pulswelle zwischen den beiden Sensoren und damit die Pulswellengeschwindigkeit zu bestimmen. Ob der Strom ansteigt oder abfällt, wenn die Sensoren in Resonanz kommen, hängt von der konkreten Ausgestaltung der Schaltung ab. Entscheidend ist die Änderung des Stromes bzw. der Impedanz.

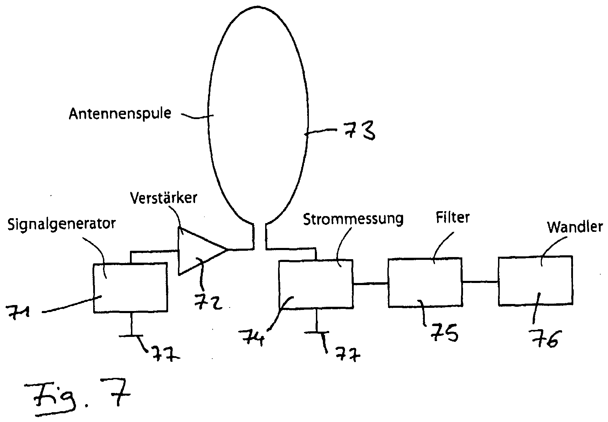

Figur 7 zeigt schematisch eine Schaltung zur Erzeugung des elektromagnetischen Wechselfeldes in der Auswerteeinheit. Wie zu erkennen ist, kann mittels eines Signalgenerators 71 eine Wechselspannung zwischen Erde 77 und einem Verstärkereingang eines Verstärkers 72 erzeugt werden. Die Wechselspannung wird vom Verstärker 72 verstärkt und liegt an einer Antennenspule 73 an. Durch die Wechselspannung durchfließt ein Wechselstrom die Antennenspule 73, der ein von der Antennenspule ausgehendes elektromagnetisches Wechselfeld verursacht. Das elektromagnetische Wechselfeld trifft auf die in der Figur 7 nicht gezeigten Schwingkreise der Sensoren des erfindungsgemäßen Pulswellengeschwindigkeitsmesssystems. Die Impedanz der Antennenspule hängt davon ab, ob die Schwingkreise der Sensoren in Resonanz sind oder nicht. Wird daher beispielsweise die Amplitude der an der Antennenspule 73 angelegten Wechselspannung konstant gehalten, so bewirkt eine Impedanzänderung eine Änderung der Stromstärke des in der Antennenspule fließenden Stromes. Wird nun mittels einer Strommessvorrichtung 74 der Stromfluss in der Antennenspule gemessen, so kann hieraus die Impedanz der Antennenspule bestimmt werden, und damit, ob einer der Schwingkreise der Sensoren in Resonanz ist. Hierzu wird das gemessene Stromsignal durch einen Tiefpassfilter 75 geführt, so dass sich an dessen Ausgang im Moment des Schneidens Stromänderungen erkennen lassen.

Mittels eines Wandlers 76 kann das analoge Signal des Filters 75 in ein digitales Signal überführt werden. Die Wandlung kann auch vor dem Filter erfolgen, so dass ein digitaler Filter zur Anwendung kommen kann.

**Patentansprüche**

1. Pulswellengeschwindigkeitsmesssystem mit einer zylinderförmigen Vorrichtung zum Durchfluss von Fluid in Richtung einer Zylinderachse der Vorrichtung,
wobei die Vorrichtung zumindest zwei Drucksensoren (3a, 3b) aufweist, die an unterschiedlichen Stellen entlang der Zylinderachse angeordnet sind und die derart ausgestaltet sind, dass mit ihnen an unterschiedlichen Stellen entlang der Zylinderachse ein Druck des Fluids im Inneren der Vorrichtung (1) messbar ist,
wobei die Drucksensoren (3a, 3b) kapazitive Drucksensoren sind, deren Kapazitäten jeweils mit zumindest einer Induktivität (12a, 12b) zu jeweils einem Schwingkreis (11a, 11b) verbunden sind, und deren Kapazitäten jeweils vom Druck des Fluids an der Stelle der Messung abhängen, wobei die jeweiligen Schwingkreise (11a, 11b) derart ausgetaltet sind, dass sie mittels zumindest eines elektromagnetischen Feldes (7) zum Schwingen anregbar sind,
sowie einer Auswerteeinheit (8), die derart ausgestaltet ist, dass mit ihr das elektromagnetische Feld (7) erzeugbar ist, mittels welchem die Schwingkreise (11a, 11b) zum Schwingen anregbar sind,
wobei die Auswerteeinheit (8) derart ausgestaltet ist, dass mit ihr das elektromagnetische Feld (7) mit Resonanzfrequenzen der Schwingkreise (11a, 11b) erzeugbar ist und der Zeitpunkt des Passierens zumindest einer Pulswelle (5a, 5b) an den zumindest zwei Drucksensoren (3a, 3b) durch Messung einer Änderung der Resonanz des jeweiligen Schwingkreises (11a, 11b) bestimmbar ist, und dass aus der Differenz der Zeitpunkte des Passierens für die zumindest zwei Drucksensoren (3a, 3b) und dem Abstand der Stellen der Messung voneinander eine Pulswellengeschwindigkeit bestimmbar ist,
wobei die Auswerteeinheit (8) derart ausgestaltet ist, dass mit ihr das elektromagnetische Feld mit zumindest einer konstanten Frequenz erzeugbar ist, die eine Resonanzfrequenz der Schwingkreise bei einem vom Normaldruck abweichenden Druck ist, und die Auswerteeinheit (8) derart ausgestaltet ist, dass mit ihr zeitabhängig bestimmbar ist, ob ein Schwingkreis resoniert, und aufeinanderfolgende Zeitpunkte des Resonierens als Zeitpunkte der Änderung der Resonanz und damit als Zeitpunkte des Passierens bestimmbar sind.

2. Pulswellengeschwindigkeitsmesssystem nach dem vorhergehenden Anspruch,
**dadurch gekennzeichnet, dass** die Drucksensoren (3a, 3b) derart ausgestaltet sind, dass mit ihnen der zeitliche Verlauf oder ein Zeitpunkt einer Druckänderung am Ort des jeweiligen Drucksensors (3a, 3b) feststellbar ist.

3. Pulswellengeschwindigkeitsmesssystem nach dem vorhergehenden Anspruch,
**dadurch gekennzeichnet, dass** das Pulswellengeschwindigkeitsmesssystem derart ausgestaltet ist, dass der zeitliche Verlauf oder der Zeitpunkt mit einer zeitlichen Auflösung feststellbar ist, die feiner ist als die Laufzeit einer Pulswelle von einem Sensor zum anderen Sensor in dem Fluid.

4. Pulswellengeschwindigkeitsmesssystem nach einem der vorhergehenden Ansprüche,
**dadurch gekennzeichnet, dass** die Schwingkreise (11a, 11b), vorzugsweise für alle zu messenden Drücke, unterschiedliche Resonanzfrequenzen aufweisen, so dass sie unabhängig voneinander zum Schwingen anregbar sind.

5. Pulswellengeschwindigkeitsmesssystem nach einem der vorhergehenden Ansprüche,
**dadurch gekennzeichnet, dass** die zylinderförmige Vorrichtung (1) ein Stent, ein Implantat, ein Katheter und/oder eine, vorzugsweise flexible, Fluidleitung ist.

6. Pulswellengeschwindigkeitsmesssystem nach einem der vorhergehenden Ansprüche, wobei die Auswerteeinheit derart ausgestaltet ist, dass mit ihr eine Impedanz messbar ist und ein Zeitpunkt der Veränderung der Impedanz bei der konstanten Frequenz als Zeitpunkt des Resonierens eins Schwingkreises und damit als Zeitpunkt des Passierens bestimmbar ist.

7. Pulswellengeschwindigkeitsmesssystem nach einem der vorhergehenden Ansprüche, **dadurch gekennzeichnet, dass** das Pulswellengeschwindigkeitsmesssystem derart ausgestaltet ist, dass das elektromagnetische Feld (7) als Puls mit einem kontinuierlichen Frequenzspektrum erzeugbar ist.

8. Verfahren zum Messen einer Pulswellengeschwindigkeit in einer Fluidleitung (2), wobei die Fluidleitung eine zylinderförmige Vorrichtung zum Durchfluss von Fluid in Richtung einer Zylinderachse der Vorrichtung aufweist,
wobei die Vorrichtung zumindest zwei Drucksensoren (3a, 3b) aufweist, die an unterschiedlichen Stellen entlang der Zylinderachse angeordnet sind und mit denen an unterschiedlichen Stellen entlang der Zylinderachse ein Druck des Fluids im Inneren der Vorrichtung (1) messbar ist,
wobei die Drucksensoren (3a, 3b) kapazitive Drucksensoren sind, deren Kapazitäten jeweils mit zumindest einer Induktivität (12a, 12b) zu jeweils einem Schwingkreis (11a, 11b) verbunden sind, und deren Kapazitäten jeweils vom Druck des Fluids an der Stelle der Messung abhängen, wobei die jeweiligen Schwingkreise (11a, 11b) mittels zumindest eines elektromagnetischen Feldes (7) zum Schwingen anregbar sind,
wobei die Schwingkreise mittels eines elektromagnetischen Feldes (7) zur Resonanz angeregt werden, wobei das elektromagnetische Feld (7) mit Resonanzfrequenzen der Schwingkreise (11a, 11b) erzeugt wird, und Zeitpunkte eines Passierens zumindest einer Pulswelle (5a, 5b) an den zumindest zwei Drucksensoren (3a, 3b) durch Messung einer Änderung der Resonanz des jeweiligen Schwingkreises (11a, 11b) bestimmt wird, und aus der Differenz der Zeitpunkte der Änderung der Resonanz für die zumindest zwei Drucksensoren (3a, 3b) und dem Abstand der Stellen der Messung voneinander die Pulswellengeschwindigkeit bestimmt wird,
wobei das elektromagnetische Feld mit zumindest einer konstanten Frequenz erzeugt wird, die eine Resonanzfrequenz der Schwingkreise bei einem vom Normaldruck abweichenden Druck ist, und zeitabhängig bestimmt wird, ob ein Schwingkreis resoniert, und aufeinanderfolgende Zeitpunkte des Resonierens als Zeitpunkte der Änderung der Resonanz und damit als Zeitpunkte des Passierens bestimmt werden.

9. Verfahren zum Messen der Elastizität einer Fluidleitung, wobei eine Pulswellengeschwindigkeit in einem Verfahren nach Anspruch 8 bestimmt wird und aus der Pulswellengeschwindigkeit die Elastizität der Fluidleitung bestimmt wird.

**10.** Vorrichtung zum Messen der Elastizität einer Fluidleitung, wobei die Vorrichtung derart ausgestaltet ist, das mit ihr eine Pulswellengeschwindigkeit in einem Verfahren nach Anspruch 8 bestimmbar ist und aus der Pulswellengeschwindigkeit die Elastizität der Fluidleitung bestimmbar ist.

**Claims**

**1.** Pulse wave velocity measurement system having a cylindrical device for the flow-through of fluid in the direction of a cylinder axis of the device,
wherein the device has at least two pressure sensors (3a, 3b) which are arranged at different locations along the cylinder axis and which are configured in such a way that a pressure of the fluid inside the device (1) is measurable by them at different locations along the cylinder axis,
wherein the pressure sensors (3a, 3b) are capacitive pressure sensors, the capacitances of which are respectively connected with at least one inductor (12a, 12b) to respectively one resonant circuit (11a, 11b), and the capacitances of which respectively depend on the pressure of the fluid at the location of the measurement, wherein the respective resonant circuits (11a, 11b) are configured in such a way that they are excitable to oscillate by means of at least one electromagnetic field (7),
and also an evaluation unit (8) which is configured in such a way that with it the electromagnetic field (7) is generatable, by means of which the resonant circuits (11a, 11b) are excitable to oscillate,
wherein the evaluation unit (8) is configured in such a way that with it the electromagnetic field (7) with resonant frequencies of the resonant circuits (11a, 11b) is generatable and the point in time when the at least one pulse wave (5a, 5b) passes the at least two pressure sensors (3a, 3b) is determinable by measuring a change of the resonance of the respective resonant circuit (11a, 11b), and that from the difference of the points in time of the passing for the at least two pressure sensors (3a, 3b) and the distance of the locations of the measurement from one another a pulse wave velocity is determinable,
wherein the evaluation unit (8) is configured in such a way that with it the electromagnetic field is generatable with at least a constant frequency which is a resonant frequency of the resonant circuits at a pressure differing from the normal pressure, and the evaluation unit (8) is configured in such a way that with it it is time-dependently determinable whether a resonant circuit resonates, and successive points in time of the resonating are determinable as points in time of the change of the resonance and thus as points in time of the passing.

**2.** Pulse wave velocity measurement system according to the preceding claim,
**characterised in that** the pressure sensors (3a, 3b) are configured in such a way that with them the time profile or a point in time of a pressure change at the location of the respective pressure sensor (3a, 3b) is ascertainable.

**3.** Pulse wave velocity measurement system according to the preceding claim,
**characterised in that** the pulse wave velocity measurement system is configured in such a way that the time profile or the point in time is ascertainable with a time resolution which is finer than the propagation time of a pulse wave from one sensor to the other sensor in the fluid.

**4.** Pulse wave velocity measurement system according to one of the preceding claims,
**characterised in that** the resonant circuits (11a, 11b), preferably for all pressures to be measured, have different resonant frequencies, so that they are excitable to oscillate independently of one another.

**5.** Pulse wave velocity measurement system according to one of the preceding claims,
**characterised in that** the cylindrical device (1) is a stent, an implant, a catheter and/or a, preferably flexible, fluid line.

**6.** Pulse wave velocity measurement system according to one of the preceding claims, wherein the evaluation unit is configured in such a way that with it an impedance is measurable and a point in time of the change of the impedance at the constant frequency is determinable as a point in time of the resonating of a resonant circuit and thus as a point in time of the passing.

**7.** Pulse wave velocity measurement system according to one of the preceding claims, **characterised in that** the pulse wave velocity measurement system is configured in such a way that the electromagnetic field (7) is generatable as a pulse with a continuous frequency spectrum.

**8.** Method for measuring a pulse wave velocity in a fluid line (2), wherein the fluid line has a cylindrical device for the flow-through of fluid in the direction of a cylinder axis of the device,

wherein the device has at least two pressure sensors (3a, 3b) which are arranged at different locations along the cylinder axis and by which a pressure of the fluid inside the device (1) is measurable at different locations along the cylinder axis,

wherein the pressure sensors (3a, 3b) are capacitive pressure sensors, the capacitances of which are respectively connected with at least one inductor (12a, 12b) to respectively one resonant circuit (11a, 11b), and the capacitances of which respectively depend on the pressure of the fluid at the location of the measurement, wherein the respective resonant circuits (11a, 11b) are excitable to oscillate by means of at least one electromagnetic field (7),

wherein the resonant circuits are excited to resonate by means of an electromagnetic field (7), wherein the electromagnetic field (7) with resonant frequencies of the resonant circuits (11a, 11b) is generated, and points in time when at least one pulse wave (5a, 5b) passes the at least two pressure sensors (3a, 3b) is determined by measuring a change of the resonance of the respective resonant circuit (11a, 11b), and from the difference of the points in time of the change of the resonance for the at least two pressure sensors (3a, 3b) and the distance of the locations of the measurement from one another the pulse wave velocity is determined,

wherein the electromagnetic field is generated with at least a constant frequency which is a resonant frequency of the resonant circuits at a pressure differing from the normal pressure, and it is time-dependently determined whether a resonant circuit resonates, and successive points in time of the resonating are determined as points in time of the change of the resonance and thus as points in time of the passing.

9. Method for measuring the elasticity of a fluid line,
wherein a pulse wave velocity is determined in a method according to claim 8 and the elasticity of the fluid line is determined from the pulse wave velocity.

10. Device for measuring the elasticity of a fluid line,
wherein the device is configured in such a way that with it a pulse wave velocity is determinable in a method according to claim 8 and the elasticity of the fluid line is determinable from the pulse wave velocity.

**Revendications**

1. Système de mesure de vitesse d'ondes d'impulsions avec un dispositif de forme cylindrique pour le passage d'un fluide en direction d'un axe de cylindre du dispositif,
le dispositif comprenant au moins deux capteurs de pression (3a, 3b) qui sont disposés à différents endroits le long de l'axe du cylindre et qui sont conçus de façon à pouvoir mesurer, à différents endroits le long de l'axe du cylindre, une pression du fluide à l'intérieur du dispositif (1),
les capteurs de pression (3a, 3b) étant des capteurs de pression capacitifs, dont les capacités sont reliées chacune avec au moins une inductance (12a, 12b) à un circuit oscillant (11a, 11b) et dont les capacités dépendant chacune de la pression du fluide à l'endroit de la mesure, les circuits oscillants (11a, 11b) étant conçus de façon à pouvoir être excités au moyen d'au moins un champ électromagnétique (7),
ainsi qu'une unité d'analyse (8), qui est conçue de façon à pouvoir générer le champ électromagnétique (7) au moyen duquel les circuits oscillants (11a, 11b) peuvent être excités,
l'unité d'analyse (8) étant conçue de façon à pouvoir générer le champ électromagnétique (7) avec des fréquences de résonance des circuits oscillants (11a, 11b) et le moment du passage d'au moins une onde d'impulsion (5a, 5b) au niveau d'au moins deux capteurs de pression (3a, 3b) peut être déterminé par la mesure d'une variation de la résonance du circuit oscillant (11a, 11b) correspondant et de façon à ce que la différence entre les moments du passage pour les au moins deux capteurs de pression (3a, 3b) et de la distance des points de mesure entre eux permette de déterminer une vitesse d'onde d'impulsion,
l'unité d'analyse (8) étant conçue de façon à pouvoir générer le champ électromagnétique avec au moins une fréquence constante, qui est une fréquence de résonance des circuits oscillants pour une pression différente de la pression normale, et l'unité d'analyse (8) étant conçue de façon à pouvoir déterminer en fonction du temps si un circuit oscillant résonne et déterminer les moments successifs de la résonance en tant que moments de variation de la résonance et donc en tant que moments du passage.

2. Système de mesure de vitesse d'ondes d'impulsions selon la revendication précédente,
**caractérisé en ce que** les capteurs de pression (3a, 3b) sont conçus de façon à déterminer l'évolution en fonction du temps ou un moment d'une variation de pression à l'endroit du capteur de pression (3a, 3b) correspondant.

3. Système de mesure de vitesse d'ondes d'impulsions selon la revendication précédente,
**caractérisé en ce que** le système de mesure de vitesse d'ondes d'impulsions est conçu de façon à pouvoir déterminer l'évolution en fonction du temps ou le moment avec une résolution temporelle qui est plus précise que le temps de passage d'une onde d'impulsion d'un capteur à l'autre dans le fluide.

4. Système de mesure de vitesse d'ondes d'impulsions selon l'une des revendications précédentes, **caractérisé en ce que** les circuits oscillants (11a, 11b) présentent, de préférence pour toutes les pressions à mesurer, des fréquences de résonance différentes, de façon à pouvoir être excités indépendamment les uns des autres.

5. Système de mesure de vitesse d'ondes d'impulsions selon l'une des revendications précédentes, **caractérisé en ce que** le dispositif de forme cylindrique (1) est un stent, un implant, un cathéter et/ou une conduite de fluide, de préférence flexible.

6. Système de mesure de vitesse d'ondes d'impulsions selon l'une des revendications précédentes, l'unité d'analyse étant conçue de façon à pouvoir mesurer une impédance et un moment de variation de l'impédance à la fréquence constante en tant que moment de la résonance d'un circuit oscillant et donc en tant que moment de passage.

7. Système de mesure de vitesse d'ondes d'impulsions selon l'une des revendications précédentes, **caractérisé en ce que** le système de mesure de vitesse d'ondes d'impulsions est conçu de façon à pouvoir générer le champ électromagnétique (7) en tant qu'impulsion avec un spectre de fréquence continu.

8. Procédé de mesure d'une vitesse d'onde d'impulsion avec une conduite de fluide (2), la conduite de fluide comprenant un dispositif de forme cylindrique pour le passage d'un fluide en direction d'un axe du cylindre du dispositif, le dispositif comprenant au moins deux capteurs de pression (3a, 3b) qui sont disposés à différents endroits le long de l'axe du cylindre et qui permettent de mesurer, à différents endroits le long de l'axe du cylindre, une pression du fluide à l'intérieur du dispositif (1), les capteurs de pression (3a, 3b) étant des capteurs de pression capacitifs, dont les capacités sont reliées chacune avec au moins une inductance (12a, 12b) à un circuit oscillant (11a, 11b) et dont les capacités dépendant chacune de la pression du fluide à l'endroit de la mesure, les circuits oscillants (11a, 11b) étant conçus de façon à pouvoir être excités au moyen d'au moins un champ électromagnétique (7), les circuits oscillants pouvant être excités en résonance au moyen d'un champ électromagnétique (7), le champ électromagnétique (7) étant généré avec des fréquences de résonance des circuits oscillants (11a, 11b) et le moment de passage d'au moins une onde d'impulsion (5a, 5b) au niveau d'au moins deux capteurs de pression (3a, 3b) étant déterminée par la mesure d'une variation de la résonance du circuit oscillant (11a, 11b) correspondant et la différence entre les moments de variation de la résonance pour les au moins deux capteurs de pression (3a, 3b) et la distance entre les points de mesure permettant de déterminer la vitesse de l'onde d'impulsion, le champ électromagnétique étant généré avec au moins fréquence constante qui est une fréquence de résonance des circuits oscillants pour une pression différente de la pression normale, et il est déterminé, en fonction du temps, si un circuit oscillant résonne et des moments successifs de la résonance sont déterminés en tant que moments de variation de la résonance et donc en tant que moments du passage.

9. Procédé de mesure de l'élasticité d'une conduite de fluide, une vitesse d'onde d'impulsion étant déterminée dans un procédé selon la revendication 8 et la vitesse de l'onde d'impulsion permettant de déterminer l'élasticité de la conduite de fluide.

10. Procédé de mesure de l'élasticité d'une conduite de fluide, le dispositif étant conçu de façon à pouvoir déterminer une vitesse d'onde d'impulsion dans un procédé selon la revendication 8, et la vitesse de l'onde d'impulsion permettant de déterminer l'élasticité de la conduite de fluide.

Fig. 1

Fig. 2

Fig. 3

EP 2 668 472 B1

Fig. 4

Fig. 5

Fig. 6

Antennenspule — 73

Verstärker

Signalgenerator

Strommessung    Filter

Wandler

71    72    74    77    75    76

77    77

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

* US 20050080346 A1

**In der Beschreibung aufgeführte Nicht-Patentliteratur**

* *Journal of Microelectromechanical Systems,* 2006, vol. 15 (5